# EUROPÄISCHE PATENTSCHRIFT

(11) **EP 1 863 444 B1**
(45) Veröffentlichungstag und Bekanntmachung des Hinweises auf die Patenterteilung: **09.12.2015**
(21) Anmeldenummer: 06723435.1
(22) Anmeldetag: 15.03.2006
(51) Int. Cl.: A61K 9/00

(54) **WAFER ENTHALTEND STEROIDHORMONE**
TABLET CONTAINING STEROID HORMONES
CACHET CONTENANT DES HORMONES STEROIDES

(30) Priorität: 31.03.2005 DE 102005015128
(43) Veröffentlichungstag der Anmeldung: 12.12.2007
(73) Patentinhaber: LTS Lohmann Therapie-Systeme AG, 56626 Andernach (DE)
(72) Erfinder: KRUMME, Markus, Randolph, NJ 07869 (US); RADLMAIER, Albert, 16727 Oberkrämer (DE); GENERAL, Sascha, 10178 Berlin (DE); DITTGEN, Michael, 99510 Apolda (DE); JENSEN, Keith, Clifton, NJ 07013 (US)
(74) Vertreter: Siemund, Volker
(86) Internationale Anmeldenummer: PCT/EP2006/002358
(87) Internationale Veröffentlichungsnummer: WO 2006/102990

(56) Entgegenhaltungen:
- WO-A2-00/42992
- WO-A2-03/015748
- DE-A1- 19 847 252
- US-A1- 2003 096 012
- US-B1- 6 264 981
- JAY S ET AL: "TRANSMUCOSAL DELIVERY OF TESTOSTERONE IN RABBITS USING NOVEL BI-LAYER MUCOADHESIVE WAX-FILM COMPOSITE DISKS" JOURNAL OF PHARMACEUTICAL SCIENCES, AMERICAN PHARMACEUTICAL ASSOCIATION. WASHINGTON, US, Bd. 91, Nr. 9, September 2002 (2002-09), Seiten 2016-2025, XP001125855 ISSN: 0022-3549

## Beschreibung

Die Erfindung betrifft eine pharmazeutische Zusammensetzung in Form eines filmförmigen Systems zur transmucosalen Verabreichung von Steroidhormonen.

Für Steroidhormone sind verschiedene Verabreichungsformen beschrieben worden. Neben der üblichen oralen Applikation stehen auch Implantate, Pflaster und Gele zur Verfügung. Diese Applikationsformen zielen auf eine kontinuierliche, möglichst gleichmäßig über einen längeren Zeitraum erfolgende Abgabe der Steroidhomone. Für verschiedene Anwendungen wäre jedoch eine Verabreichung wünschenswert, die zu einem schnellen Anstieg der Konzentration des Steroidhormons im Blut führt. Um z. B. bei einem Testosteronmangel beim Mann den physiologischen Zustand wiederherzustellen, müsste ein morgendlicher Konzentrationspeak erreicht werden. Voraussetzung dafür wäre eine rasche Aufnahme des Hormons und eine hohe Bioverfügbarkeit, um in kurzer Zeit eine hohe Blutkonzentration zu erreichen.

Eine rasche Freisetzung von Wirkstoffen kann durch eine transmucosale Applikation erzielt werden. Dafür sind Darreichungsformen bekannt, die in wässriger Umgebung, z. B. in der Mundhöhle, zerfallen. Bukkale Applikationssysteme wie Pflaster, Lutschtabletten, Kaugummi, Filme und schmelzende Tabletten sind bekannt.

Besonders sind hier filmförmige Systeme zu nennen, die sogenannten Wafer (US 5,948,430). Nach Applikation eines Wafers im Mundraum wird der Wirkstoff an die Umgebung freigesetzt. Um einen schnellen Konzentrationsanstieg des Wirkstoffs im Blut zu erzielen, kommt es besonders auf die schnelle Resorption des Wirkstoffs durch die Mundschleimhaut an. Eine schlechte Löslichkeit oder Resorption kann nicht durch eine Vergrößerung des Wafers beliebig ausgeglichen werden, da der Wafergröße durch die Größe der Mundhöhle Grenzen gesetzt sind und dicke Wafer nur sehr langsam zerfallen.

Die in den Wafern enthaltenen Arzneimittelwirkstoffe werden durch die Mundschleimhaut je nach Absorptionsstelle entweder bukkal oder sublingual absorbiert. Diese Verabreichungsarten zeichnen sich durch zahlreiche Vorteile gegenüber der *per oralen* Gabe aus, wie z. B. die Umgehung des First-pass-Effektes, schnellerer Wirkungseintritt sowie Vermeidung des gastrointestinalen Metabolismus.

Bei der Entwicklung eines bukkalen oder sublingualen Applikationssystems spielt die Mucoadhäsion eine Schlüsselrolle. Materialien, die zu der Mucinschicht biologischer Membrane binden, werden üblicherweise als "mucoadhäsiv" bezeichnet. Mucoadhäsive Polymere wurden mehrfach in zahlreichen Applikationsformen eingesetzt, um bestimmte Wirkstoffe durch Gabe über verschiedene Schleimhäute systemisch bioverfügbar zu machen. Tabletten, Pflaster, Streifen, Filme, Semisolide und Pulver zählen zu solchen Arzneimittelformulierungen Polymere müssen bestimmte physikochemische Eigenschaften besitzen, um mucoadhäsiv zu sein. So müssen solche Polymere durch zahlreiche wasserstoffbrückenbildende Gruppen überwiegend anionisch hydrophil sein, eine ausreichende Benetzbarkeit auf der Schleimhautgewebeoberfläche sowie ausreichende Flexibilität aufweisen, um durch die Schleimhaut bzw. Gewebespalte zu penetrieren.

Filmförmige Formulierungen zur mukosalen Verabreichung von Wirkstoffen, z.B. Estradiol, werden in WO 00/42 992 A offenbart. Die Formulierungen werden durch ein Trockenextrudierverfahren hergestellt. WO 03/015 748 A beschreibt mehrschichtige mukoadhäsive Darreichungsformen zur mukosalen Freisetzung von Wirkstoffen.

Ein Hauptproblem bei der Entwicklung bukkaler und sublingualer Applikationssysteme ist jedoch die niedrige Wirkstoffflussrate durch das mucosale Epithelgewebe, welche zu einer niedrigen Bioverfügbarkeit des Wirkstoffes führt.

Die Fähigkeit eines Arzneistoffs, in die menschliche orale Mucosa einzudringen, hängt unter anderem von der Fettlöslichkeit des Arzneistoffs ab, die durch den Öl/Wasser-Verteilungskoeffizienten ausgedrückt wird. Für Carbonsäuren, AlkylPhenyl-Essigsäuren, Fettsäuren, Amphetamine und Fenfluramine, Acetanilide und Steroide wurde dieser Zusammenhang beispielhaft gezeigt.

Für Steroide wurde gezeigt, dass deren bukkale Absorption in einer biexponentiellen Funktion von den Öl/Wasser-Verteilungskoeffizienten abhängt. Für die sublinguale Absorption werden log P-Werte zwischen 1,6 und 3,3 als günstig angesehen. Für eine Reihe von Progesteronderivaten konnte gezeigt werden, dass mit abnehmendem log P (zunehmender Hydrophilie) die Geschwindigkeitskonstante für die Aufnahme durch die Schleimhaut abnimmt.

Da zwei parallele Wege der bukkalen Absorption bestehen, wurde postuliert, dass Substanzen mit nahezu gleicher Löslichkeit in Wasser und Öl am besten permeieren. Dem wurde jedoch der Befund gegenüber gestellt, dass in homologen Reihen die Permeation mit zunehmender Hydrophobizität zunimmt.

Weitere Parameter zur Beurteilung der Absorption von Wirkstoffen durch die bukkale Mucosa sind physikochemische Eigenschaften wie die Löslichkeit und die Lösungsgeschwindigkeit. Die Löslichkeit eines Wirkstoffes in dem im Mundraum vorliegenden Medium bestimmt den Konzentrationsgradienten, der den Diffusionsdruck beschreibt. Eine hohe Löslichkeit erzeugt einen hohen Diffusionsdruck. Dabei beträgt das vorhandene Flüssigkeitsvolumen im Mundraum nur wenige Milliliter.

Die hier beschriebenen Steroidhormone zeigen eine Löslichkeit von 30 µg bis maximal 1 mg in dem angebotenen Volumen. Die für eine medizinische Anwendung erforderliche Menge liegt bei allen Substanzen jedoch deutlich darüber. US 6,264,981 beschreibt verschiedene Möglichkeiten, die diese Herausforderung annehmen. Bei schwachen Säuren und Basen, also ionisierbaren Substanzen, wird in US 6,264,981 eine gepufferte Formulierung beschrieben. Die Pufferung führt dazu, dass die dort genannten Substanzen in einer ionisierten und damit besser löslichen Salzform vorliegen. Die hier beschriebenen Steroidhormone sind jedoch nicht ohne Weiteres in eine Salzform überführbar.

Verschiedene chemische Substanzen wurden auf ihre Einsetzbarkeit als Penetrations- und Absorptionsverstärker bei transmucosaler Anwendung getestet, wobei die Verträglichkeit und Sicherheit eine wichtige Rolle spielte. In der für die Steigerung der mucosalen Absorption erforderlichen Konzentration führte die Verwendung vieler bekannter Permeationsverstärker jedoch potentiell zur Irritation und/oder Beschädigung der mucosalen Membrane.

Da Permeationsverstärker "per definitionem" die Schleimhaut schädigen, zählen zu den schwerwiegenden Nachteilen der Permeationsverstärkung mit derartigen Permeationsverstärkern die Zerstörung der bukkalen Schleimhaut beispielsweise durch Verlust der oberen Zellschichten und der Abnahme der Zahl der Desmosomen, sowie die Irritation des bukkalen Epithels durch Salze, Natriumlaurylsulfat oder Gallensäuren.

Steroidhormone sind lipophile Substanzen, die nur sehr schlecht wasserlöslich sind. Im Angesicht der metabolischen Clearance von Testosteron (C. Wang, D. H. Catlin, B. Starcevic, A. Leung, E. DiStefano, G. Lucas, J. Clin. Endocrin. Metab., 89, 2936-2941, 2004) und der pharmakokinetischen Daten für die dem Mann buccal applizierte Testosteron-Tablette, errechnet sich für die bukkaladhäsive Testosteron-Tablette ein Wert für Bioverfügbarkeit in der Größenordnung von 25 % (K. J. Baisley, M. J. Boyce, S. Bukofzer, R. Pradhan, S. J. Warrington, J. Endocrin. 175, 813-819, 2002).

Da filmförmige Applikationssysteme für eine bukkale Anwendung sowohl in der Fläche als auch in der Dicke beschränkt sind, ist eine Bioverfügbarkeit von 25 % für einige Steroide nicht ausreichend. Die Fläche des Wafers ist begrenzt durch die freie bukkale Fläche, die ca. 7 cm² je Seite beträgt. Wird der Wafer größer ist eine gleichförmige und sichere Applikation nur mit verminderter Zuverlässigkeit zu gewährleisten. Es ist auch nicht ohne weiteres möglich, durch eine Erhöhung der Beladung der Wafer oder durch übermäßige "Verdickung" des Wafers eine höhere Konzentration an gelöstem Wirkstoff an der Resorptionsstelle (bukkale Mucosa) zu erzielen, da hierdurch das Risiko eines ungewollten Schluckens des Wirkstoffs durch neugebildeten Speichel zunimmt.

Für eine sehr schnelle und hohe Resorption ist man daher an Filme gebunden, die sowohl das Trägermaterial als auch den Wirkstoff in sehr kurzer Zeit (vorzugsweise innerhalb von weniger als 15 min) auflösen und damit zur Resorption durch die bukkale Mucosa bereitstellen. Auch ist es für einen schnellen Wirkungseintritt nicht zweckdienlich, eine Folge von bukkal applizierten Filmen mit geringen Verfügbarkeiten anzuwenden. Die Wiederherstellung der Bedingungen im Mundraum wie vor Anwendung des ersten Filmes innerhalb einer solchen Reihe würde den kompletten Austausch des vorhandenen Speichels erfordern. Außerdem muss eine vollständige Resorption oder Entfernung der vorherigen Wafers garantiert sein, um eine konstante und damit reproduzierbare Resorption zu gewährleisten. Diese Vorgaben hätten einen zeitlichen Abstand zwischen den Anwendungen zweier Wafer von mindestens 30 min zur Folge.

Die Löslichkeit von Steroidhormonen, die in physiologischen pH-Bereichen nicht ionisierbar sind und die mit einem log P-Wert von 1,0 - 4,3 nicht ausreichend löslich sind, lässt sich bei gleicher Resorption auch nicht durch die in US 6,264,981 beschriebenen anderen Maßnahmen (Cyclodextrine, Einschlussverbindungen) verbessern.

Die Lösungsgeschwindigkeit von Trägermaterial und Wirkstoff ist speziell für die Anwendung im Mundraum von großer Bedeutung, da dort ständig etwa 0,5 - 3 ml/min frischer Speichel gebildet wird. Dieser wird anschließend, durch den Anwender nicht kontrollierbar, durch Verschlucken in den Magen-Darm-Trakt befördert. Dort unterliegt der Wirkstoff, wie bei oraler Gabe, den Nachteilen einer langsamen Aufnahme und des metabolischen First-Pass-Effektes.

Die Aufgabe der vorliegenden Erfindung lag daher darin, ein sich in der Mundhöhle auflösendes Applikationssystem für bei physiologischen pH-Werten nicht ionisierbaren Steroidhormonen mit einem log P Wert von 1,0 - 4,3 vorzugsweise ohne zusätzliche Permeationsverstärker zur Verfügung zu stellen, das die darin enthaltenen Steroidhormone mit einer hohen Bioverfügbarkeit, vorzugsweise größer als 50 %, freisetzt, wobei der maximale Blutspiegel (= maximale Konzentration) des darin enthaltenen Steroidhormons innerhalb von 60 Minuten erreicht wird.

Die der vorliegenden Erfindung zugrunde liegende Aufgabe wurde durch die Bereitstellung eines sich in der Mundhöhle auflösenden Applikationssystems gelöst, das 5 Gew.-% eines Steroidhormons und 95 Gew.-% eines Trägermaterials enthält.

Gegenstand der Erfindung ist ein Applikationssystem, das 5 Gew.% eines Steroidhormons aus der Gruppe bestehend aus Testosteron, 7α-Methyl-19-nortestosteron und 7α-Methyl-11β-fluor-19-nortestosteron und 95 Gew.-% Hydroxypropylmethylcellulose (HPMC) enthält.

Bevorzugt gemäß vorliegender Erfindung sind die sich in der Mundhöhle auflösenden Applikationssysteme filmförmig. Diese filmförmigen Applikationssysteme werden auch als "Wafer" bezeichnet. Die erfindungsgemäßen filmförmigen Applikationssysteme können in einer besonderen Ausführungsform mucoadhäsiv ausgerüstet sein. Darunter ist die Eigenschaft zu verstehen, auf einer Schleimhaut zu haften, und zwar in einer Weise, dass nach der Applikation ein anschließendes Abziehen des Applikationssystems von der Schleimhaut unmöglich ist.

Die filmförmigen Applikationssysteme der vorliegenden Erfindung besitzen eine Fläche zwischen 1 und 10 cm², vorzugsweise zwischen 5 und 8 cm² und besonders bevozugt von 7 cm². Dabei besitzen sie ein Flächengewicht zwischen 50 und 250 g/m², vorzugsweise zwischen 100 und 150 g/m². Letzteres korreliert in etwa mit einer Dicke zwischen 40 und 130 µm, vorzugsweise zwischen 50 und 100 µm.

Das filmförmige Applikationssystem löst sich in der Mundhöhle in einem Zeitraum von weniger als 30 min, besonders bevorzugt in einem Zeitraum von weniger als 15 min auf. Das aus dem Applikationssystem transmucosal in den Blutkreislauf eintretende Steroidhormon führt zu einem schnellem Anstieg der Konzentration dieses Steroidhormons im Blut. Dabei ist ein Maximum der Konzentration dieses Steroidhormons im Blut vorzugsweise in einem Zeitraum von weniger als 60 min - besonders bevorzugt in einem Zeitraum zwischen 15 und 30 min - nach Applikation erreicht.

Charakteristisch für das erfindungsgemäße Applikationssystem ist die hohe Bioverfügbarkeit des Steroidhormons und die Fähigkeit, einen pulsförmigen Verlauf der Hormonkonzentration im Blut zu erzielen mit einem deutlichen Abklingen. Das Applikationssystem ermöglicht so eine pulsatile, circadiane oder ähnliche, dem natürlichen Rhythmus angepasste Hormontherapie.

Es kann mit dem Applikationssystem eine Bioverfügbarkeit der Steroidhormone von mindestens 25 % erreicht werden, vorzugsweise von mindestens 50 %. In einer besonders bevorzugten Ausführungsform wird das Steroidhormon mit einer Bioverfügbarkeit zwischen 70 und 75 % freigesetzt.

Das filmförmige Applikationssystem kann neben dem Trägermaterial und dem Steroidhormon weitere Stoffe enthalten, beispielsweise Geschmacksstoffe, Farbstoffe, Permeationsverstärker, Süßstoffe, Füllstoffe, flüssige - vorzugsweise lipophile - Hilfsstoffe, die befähigt sind, das Steroidhormon zu lösen und eine zweite Phase in dem - vorzugsweise hydrophilen - Trägermaterial zu bilden, Lösungsvermittler, pH-Stabilisatoren, Sprengmittel. In einer bevorzugten Ausführungsform ist das Applikationssystem frei von Penetrationsverstärkern, Absorptionsverstärkern und / oder Permeationsverstärken.

Bevorzugt wird das Applikationssystem zur pulsatilen Behandlung im circardianen Rhythmus eingesetzt. Eine pulsatile Freisetzung des Arzneistoffs ermöglicht im allgemeinen eine bessere (chronopharmazeutische) Behandlung von Krankheiten mit einem oszillatorischen Rhythmus in ihrer Pathogenese, wie Asthma, Arthritis, Dünndarmkrebs u. a. Krebserkrankungen, Diabetes, Herz-Kreislauferkrankungen, Erkrankungen der Gallenblase und neurologische Erkrankungen.

Besonders bevorzugt wird das Applikationssystem im Rahmen einer Therapie verwendet, bei der mittels einer einmal-täglichen Verabreichung in kurzer Zeit, vorzugsweise in einem Zeitraum von weniger als 60 min eine erhöhte Konzentration eines Steroidhormons im Blut erzielt wird. Das Applikationssystem kann insbesondere im Rahmen einer Androgentherapie einmal morgens verabreicht werden, um in kurzer Zeit eine erhöhte Blutkonzentration des verabreichten Androgens zu erzielen.

Steroidhormone gemäß vorliegender Erfindung können Androgene mit einem log P-Wert von 1,0 - 4,3 sein.

Im Sinne der vorliegenden Erfindung geeignete Androgene sind Testosteron, 7α-Methyl-19-nortestosteron (MENT) und 7α-Methyl-11ß-fluor-19-nortestosteron (eF-MENT).

Sämtliche Beispiele fallen in den log P-Bereich von 1,0 - 4,3.

Bevorzugt sind gemäß vorliegender Erfindung Applikationssysteme enthaltend das Androgen MENT oder eF-MENT.

Besonders bevorzugt sind Applikationssysteme, die 95 % HPMC und 5 % MENT oder eF-MENT enthalten.

Die folgenden Beispiele erläutern die Herstellung geeigneter Applikationssysteme ("Wafer"). In Tabelle 1 sind die Zusammensetzungen der getrockneten Wafer wiedergegeben.

Beispiel 1: 5 g MENT werden zu 700 ml einer Lösung von Ethanol/Wasser (50:50) gegeben und bis zum vollständigen Auflösen gerührt. Gegebenenfalls wird das Auflösen durch Anwendung von Ultraschall unterstüzt. Anschließend wird 95 g Hydroxypropylmethylcellulose (HPMC) dazugegeben und bis zur vollständigen Auflösung gerührt. Die Mischung wird entgast, mit Hilfe eines Streichkastens ausgestrichen und getrocknet. Es entsteht ein dünner, transparenter Film, der zwischen 50 und 100 µm dick ist. Durch Auschneiden von Proben entsprechender Größe erhält man transparente Wafer mit einem Gehalt von 1,5 mg MENT.

Vergleichsbeispiel 2: 3 g Menthol und 2 g Thymol werden zu 26 g ATMOS 300 (= eine Mischung von Mono- und Diglyceriden der Ölsäure) und 4 g Tween 80 (= ein Polyoxyethylen-Sorbitanoleat-Ester) gegeben. Es wird gerührt, bis sich die Feststoffe gelöst haben. Anschließend werden 5 g MENT zu dieser Mischung gegeben und bis zum vollständigen Lösen des Wirkstoffs gerührt. 60 g HPMC werden zu 600 g einer 50:50-Ethanol-Wasser-Mischung gegeben und bis zur vollständigen Auflösung gerührt. Die organische Phase wird nun langsam und unter schnellem Rühren zur wässrigen Phase gegeben, wobei eine dicke, cremige Masse entsteht. Nach Entgasen der Mischung und Ausstreichen wird getrocknet. Es entsteht ein dünner durchscheinender Film, der zwischen 50 und 100 µm dick ist. Durch Auschneiden von Proben entsprechender Größe erhält man durchscheinende Wafer mit einem Gehalt von 1,5 mg MENT.

Vergleichsbeispiel 3: 5 g MENT werden zu einer Mischung von 20 g ATMOS 300, 10 g Octanol und 5 g Lecithin gegeben. Es wird gerührt, bis sich die Feststoffe gelöst haben. 60 g HPMC werden zu 600 g Wasser gegeben und bis zur Auflösung gerührt. Die organische Phase wird unter schnellem Rühren zur wässrigen Phase gegeben, wobei eine dicke, cremige Masse entsteht. Nach Entgasen der Mischung und Ausstreichen wird getrocknet. Es entsteht ein dünner durchscheinender Film, der zwischen 50 und 100 µm dick ist. Durch Auschneiden von Proben entsprechender Größe erhält man durchscheinende Wafer mit einem Gehalt von 1,5 mg MENT.

**Tab. 1: Zusammensetzungen getrockneter MENT-Wafer**

| **Beispiel** | **MENT (%)** | **HPMC (%)** | **ATMOS 300 (%)** | **Tween 80 (%)** | **Octanol (%)** | **Lecithin (%)** | **Menthol (%)** | **Thymol (%)** |
|---|---|---|---|---|---|---|---|---|
| 1 | 5 | 95 | - | - | - | - | - | - |
| 2 | 5 | 60 | 26 | 4 | - | - | 3 | 2 |
| 3 | 5 | 60 | 20 | - | 10 | 5 | - | - |

Es sei angemerkt, dass die getrockneten Systeme gemäß Beispiel 1 das Steroidhormon monomolekular dispers gelöst in dem Trägermaterial enthalten, während in den Vergleichsbeispielen 2 und 3 das Steroidhormon als Lösung in einer Ölphase vorliegt, die wiederum in dem Trägermaterial als separate Phase enthalten ist. Durch die Verwendung von flüssigen, vorzugsweise lipophilen Hilfsstoffen, die befähigt sind, das Steroidhormon zu lösen und eine zweite Phase in dem (vorzugsweise hydrophilen) Trägermaterial zu bilden, können so filmförmige Applikationssysteme mit Steroidhormonen als "Zweiphasensystem" hergestellt werden.

### Experimentelle Daten

In einer klinischen Pilotstudie wurde an gesunden Männern geprüft, ob ein MENT-Wafer prinzipiell für den klinischen Einsatz geeignet ist. Wesentliche Aspekte der Prüfung betrafen die durch den Wafer erzielbaren Wirkstoffspiegel im Blut und deren zeitlichen Verlauf, sowie die allgemeine Verträglichkeit. Es wurden drei verschiedene Dosierungen des Wafers geprüft. Jeder Proband erhielt in einem cross-over design alle drei Dosierungen jeweils als Einmalgabe. Zwischen den Einmalgaben lag eine Wash-out phase von mindestens 48 Stunden.

Die drei Dosierungen wurden so festgelegt, dass eine realistische Möglichkeit bestand, MENT-Blutspiegel mit der vorhandenen Messmethodik auch bei sehr geringer Bioverfügbarkeit zumindest in der hohen Dosisstufe noch messen zu können und andererseits bei sehr hoher Bioverfügbarkeit theoretisch denkbare Spitzenwerte sich noch innerhalb des mit klinischen oder präklinischen Daten belegten Sicherheitsbereiches befinden.

Die Dosisgruppen waren 0.5 mg, 1.5 mg und 3.0 mg. Die Höhe der Dosierung wurde mit der Größe des Wafers bestimmt. Der verwendete Wafer enthielt 0.225 mg MENT / 1 cm². Die Einmalgabe erfolgte in der 0.5 mg Dosis mit einem Wafer der Fläche 2.22 cm² (Formulierung a), bei 1.5 mg mit einem Wafer der Fläche 6.67 cm² (Formulierung b), und bei 3.0 mg mit zwei Wafern von je 6.67 cm². Die Wafer wurden vom Prüfarzt auf die Buccalschleimhaut appliziert. Die niedrige Dosis wurde immer auf der rechten Seite eingebracht, die mittlere immer auf der linke Seite und für die hohe Dosis wurde auf jeder Seite ein Wafer verwendet.

Insgesamt erhielten 11 Männer im Alter zwischen 23 und 42 Jahren die Wafer, ohne dass relevante Nebenwirkungen beobachtet wurden. Die lokale Verträglichkeit war sehr gut. Eine visuelle Inspektion der Applikationsstelle ergab keine Anzeichen für unerwünschte Lokalreaktionen. Die Probanden gaben ihren subjektiven Eindruck zur lokalen Verträglichkeit unter Verwendung einer Visuellen Analogskala wieder. Auch hier ergab sich kein Hinweis für relevante unerwünschte Effekte.

Der Wafer war bei der Mehrzahl der Anwendungen innerhalb von 15 Minuten aufgelöst. In einigen Fällen dauerte es länger; die maximale Dauer lag in einem Fall bei 33 Minuten. Die verfügbaren Daten unterstützen die Annahme, dass im Falle einer deutlich verlängerten Zeit bis zur Auflösung des Wafers die Bioverfügbarkeit geringer ist.

Der MENT Wafer zeigte eine überraschend gute Bioverfügbarkeit von ca. 70 - 75 % (Tab. 2).

**Tab. 2: Bioverfügbarkeit des MENT-Wafers**

| | | | AUC(0-tlast) | AUC(0-tlast) | AUC(0-tlast) | **Bioverfügbarkeit** |
|---|---|---|---|---|---|---|
| Behandlung | Formulierung | | | | | |
| | | Dosis | geomean | geomean *Dosisnormiert* | geomean *Dosisnormiert* | **[%]** |
| | | (mg) | [ng x h/ml] | [ng x h/ml] | [ng x h/ml] | |
| A | a | 0,50 | 3,78 | 3,78 | | **75,9** |
| B | b | 1,50 | 11,00 | 3,67 | 4,98 | **73,6** |
| C | b | 3,00 | 21,10 | 3,52 | *(nach i.v. Gabe von 0,5 mg MENT)* | **70,6** |

Die interindividuelle Variation der Gesamtexposition ist mit unter 20 % als gering einzustufen.

Sowohl C-max als auch Gesamtexposition zeigen eine deutliche Dosis-Linearität. Die höchsten Konzentrationen werden binnen 15 bis 30 Minuten gemessen, fallen dann schnell ab; nach mehr als 4 Stunden sind nur noch unbedeutende Konzentrationen messbar.

Für die Bestimmung der MENT-Serumspiegel wurde eine (speziell für diesen Zweck entwickelte und validierte) GC-MS-Methode (Gaschromatographie-Massenspektrometrie-Kupplung) eingesetzt. Die Methode bedient sich des Verfahrens der negativen chemischen Ionisierung (NCI = negative chemical ionisation) und erreicht dadurch eine hohe Sensitivität, die es erlaubt, Konzentrationen bis zu einer unteren Grenze von ungefähr 60 pg/ml zu messen (lower limit of quantification).

Für Messungen mit geringerer Sensitivität steht auch eine GC-MS Methode bereit, die anstelle der NCI das Verfahren der Elektronen-Ionisierung (EI = electron ionisation) einsetzt.

Prinzipiell kann die MENT-Konzentration auch mit anderen Verfahren bestimmt werden, die ansonsten für die Bestimmung von Steroidhormonen in Frage kommen. Als geeignete Beispiele ohne Anspruch auf Vollständigkeit sind zu nennen Radioimmunassays, LC-MS Techniken, oder HPLC-Verfahren (High Performance Liquid Chromatography).

**Tab. 3 Übersicht zu pharmakokinetischen Kenndaten der getesteten Dosierungen des MENT Wafers**

| | | | Cmax | Tmax | AUC(0-tlast) |
|---|---|---|---|---|---|
| Behandlung | Formulierung | Dosis | geomean (CV) | median (range) | geomean (CV) |
| | | (mg) | [ng/ml] | [h] | [ng x h/ml] |
| A | a | 0,50 | 3.29 (67.8%) | 0.25 | 3.78 |
| | | | | (0.25-2.00) | (44.5%) |
| B | b | 1,50 | 8.98 (39.4%) | 0.50 | 11.0 |
| | | | | (0.25-0.75) | (17.8%) |
| C | b | 3,00 | 18.2 (31.2%) | 0.50 | 21.1 |
| | | | | (0.25-0.75) | (16.2%) |

| | | | | | |
|---|---|---|---|---|---|
| CV = Variationskoeffizient | | | | | |

Vorliegende klinische Ergebnisse mit MENT weisen darauf hin, dass für eine wirksame Verhinderung hypogonadaler Beschwerden bei Männern Serumkonzentrationen von mindestens 0.3 ng / ml (ca. 1 nmol/l) erforderlich sind. Dies ist etwa 10-fach niedriger als die Mindestkonzentration von Testosteron.

Das MENT-Wafer-System erreicht diese Serumspiegel problemlos. Mit der vorgefundenen Pharmakokinetik reicht der MENT-Wafer an eine intravenöse Gabe heran. Wegen der kurzen Halbwertszeit ist der Wafer dort sinnvoll einzusetzen, wo eine kurzzeitige, aber sehr effektive Androgenanflutung wünschenswert ist. Als "kurzzeitig" ist bei dieser Indikation ein Zeitraum von weniger als 60 min, vorzugsweise zwischen 15 und 30 min anzusehen.

Ein Vorteil der Anwendung, die sich auf den akuten Bedarfsfall beschränkt, ist darin zu sehen, dass die Hemmwirkung auf die Funktion der Hoden nur gering ist und mit keiner wesentlichen Beeinträchtigung der entsprechenden physiologischen Funktionen zu rechnen ist. Im Gegensatz zu den länger wirksamen Androgenprodukten ist daher bei Verwendung des erfindungsgemäßen Applikationssystems mit einem Androgen als Wirkstoff weder mit einer Beeinträchtigung der vorhandene gonadalen Testosteronsynthese noch mit der Hemmung der Spermatogenese zu rechnen.

Als Einsatzbereich für das erfindungsgemäße Applikationssystem aus dem Bereich der Androgene kommt daher die einmal tägliche Applikation zur Wiederherstellung der circadianen Androgenrhythmik bei älteren Männer. Die bei Männern mit dem Alter einhergehende Absenkung der endogenen Testosteronkonzentration ist vor allem dadurch gekennzeichnet, dass die circadiane Rhythmik verloren geht. Der in den Morgenstunden zu beobachtende Anstieg der Testosteronspiegel entfällt weitgehend. Dadurch ist der größte Unterschied in den Testosteronspiegeln zwischen jungen und alten Männern in den morgendlichen Blutabnahmen nachweisbar, wogegen bei einer Abnahme am Abend nur geringe Unterschiede bestehen. Mit Hilfe des erfindungsgemäßen Applikationssystems kann der altersbedingte relative Androgenmangel entsprechend der Androgenrhythmik behandelt werden, wobei die vorhandene körpereigene Testosteronproduktion praktisch kaum beeinträchtigt wird. Dabei kann in vorteilhafter Weise die Behandlung vom Patienten selbst einfach und bequem durchgeführt werden.

## Patentansprüche

1. Filmförmiges Applikationssystem für die transmucosale Verabreichung eines Steroidhormons, umfassend 5 Gew.-% eines Steroidhormons aus der Gruppe bestehend aus Testosteron, 7α-Methyl-19-nortestosteron und 7α-Methyl-11β-fluor-19-nortestosteron und 95 Gew.-% Hydroxypropylmethylcellulose (HPMC), wobei das Steroidhormon in dem Trägermaterial gelöst vorliegt, **dadurch gekennzeichnet, dass** es sich in einem Zeitraum von weniger als 30 Minuten in der Mundhöhle vollständig auflöst.

2. Applikationssystem nach einem der vorangehenden Ansprüche, **dadurch gekennzeichnet, dass** es eine Fläche zwischen 1 und 10 cm², vorzugsweise eine Fläche zwischen 5 und 8 cm² und besonders bevorzugt eine Fläche von 7 cm² besitzt.

3. Applikationssystem nach einem der vorangehenden Ansprüche, **dadurch gekennzeichnet, dass** es ein Flächengewicht zwischen 50 und 250 g/m², vorzugsweise zwischen 100 und 150 g/m² besitzt.

4. Applikationssystem nach einem der vorangehenden Ansprüche, **dadurch gekennzeichnet, dass** es eine Dicke zwischen 40 und 130 µm, vorzugsweise zwischen 50 und 100 µm besitzt.

5. Applikationssystem nach einem der vorangehenden Ansprüche, **dadurch gekennzeichnet, dass** es mucoadhäsiv ist.

6. Applikationssystem nach einem der vorangehenden Ansprüche, **dadurch gekennzeichnet, dass** es das darin enthaltene Steroidhormon bei bukkaler Applikation mit einer Bioverfügbarkeit zwischen 70 und 75% freisetzt.

7. Verfahren zur Herstellung eines filmförmigen Applikationssystems für die transmucosale Verabreichung eines Steroidhormons aus der Gruppe bestehend aus Testosteron, 7α-Methyl-19-nortestosteron und 7α-Methyl-11β-fluor-19-nortestosteron, **dadurch gekennzeichnet, dass** das Steroidhormon in Form einer Lösung in einem pharmazeutisch unbedenklichen Lösungsmittel mit einer wasserhaltigen Mischung eines Trägermaterials, wobei das Trägermaterial Hydroxypropylmethylcellulose (HPMC) ist, gemischt wird, die dabei entstehende Mischung als eine dünne Schicht ausgestrichen wird und durch Entfernung des Lösungsmittels und unter Bildung eines Films getrocknet wird.

8. Verfahren nach Anspruch 7, **dadurch gekennzeichnet, dass** der entstandene Film durch Perforieren, Querschneiden und / oder Längsschneiden in einzelne Applikationssysteme unterteilt wird.

## Claims

1. Administration system in film form for transmucosal administration of a steroid hormone, comprising 5% by weight of a steroid hormone from the group consisting of testosterone, 7α-methyl-19-nortestosterone and 7α-methyl-11β-fluoro-19-nortestosterone and 95% by weight of hydroxypropylmethylcellulose (HPMC), wherein the steroid hormone is present dissolved in the carrier material, **characterized in that** it dissolves completely in a period of less than 30 minutes in the mouth.

2. Administration system according to Claim 1, **characterized in that** it has an area of between 1 and 10 cm², preferably an area of between 5 and 8 cm² and particularly preferably an area of 7 cm².

3. Administration system according to either of the preceding claims, **characterized in that** it has a weight per unit area of between 50 and 250 g/m², preferably between 100 and 150 g/m².

4. Administration system according to any of the preceding claims, **characterized in that** it has a thickness of between 40 and 130 µm, preferably between 50 and 100 µm.

5. Administration system according to any of the preceding claims, **characterized in that** it is mucoadhesive.

6. Administration system according to any of the preceding claims, **characterized in that** it releases the steroid hormone contained therein on buccal application with a bioavailability of between 70 and 75%.

7. Process for producing an administration system in film form for transmucosal administration of a steroid hormone from the group consisting of testosterone, 7α-methyl-19-nortestosterone and 7α-methyl-11β-fluoro-19-nortestosterone, **characterized in that** the steroid hormone is mixed in the form of a solution in a pharmaceutically acceptable solvent with a water-containing mixture of a carrier material, where the carrier material is hydroxypropylmethylcellulose (HPMC), the mixture produced thereby is spread out as a thin layer and is dried by removing the solvent and with formation of a film.

8. Process according to Claim 7, **characterized in that** the resulting film is divided into individual administration systems by perforation, cutting transversely and/or cutting longitudinally.

## Revendications

1. Système d'administration pelliculaire pour l'administration transmucosale d'une hormone stéroïdienne, comprenant 5 % en poids d'une hormone stéroïdienne choisie dans le groupe constitué par la testostérone, la 7α-méthyl-19-nortestostérone et la 7α-méthyl-11β-fluoro-19-nortestostérone et 95 % en poids d'hydroxypropylméthylcellulose (HPMC), l'hormone stéroïdienne étant présente en solution dans le matériau de support, **caractérisé en ce qu'**il se dissout totalement dans la cavité buccale en un espace de temps de moins de 30 minutes.

2. Système d'administration selon la revendication 1, **caractérisé en ce qu'**il présente une surface comprise entre 1 et 10 cm², de préférence une surface comprise entre 5 et 8 cm² et de façon particulièrement préférée une surface de 7 cm².

3. Système d'administration selon l'une quelconque des revendications précédentes, **caractérisé en ce qu'**il présente un poids par unité de surface compris entre 50 et 250 g/m², de préférence entre 100 et 150 g/m².

4. Système d'administration selon l'une quelconque des revendications précédentes, **caractérisé en ce qu'**il présente une épaisseur comprise entre 40 et 130 µm, de préférence entre 50 et 100 µm.

5. Système d'administration selon l'une quelconque des revendications précédentes, **caractérisé en ce qu'**il est mucoadhésif.

6. Système d'administration selon l'une quelconque des revendications précédentes, **caractérisé en ce que** lors de l'administration buccale il libère l'hormone stéroidienne qu'il contient avec une biodisponibilité comprise entre 70 et 75 %.

7. Procédé pour la fabrication d'un système d'administration pelliculaire pour l'administration transmucosale d'une hormone stéroïdienne choisie dans le groupe constitué par la testostérone, la 7α-méthyl-19-nortestostérone et la 7α-méthyl-11β-fluoro-19-nortestostérone, **caractérisé en ce qu'**on mélange l'hormoné stéroïdienne sous forme d'une solution dans un solvant pharmaceutiquement acceptable avec un mélange aqueux d'une substance véhicule, la substance véhicule étant l'hydroxypropylméthylcellulose (HPMC), on étale sous forme d'une mince couche le mélange qui en résulte et on le sèche par élimination du solvant et avec formation d'un film.

8. Procédé selon la revendication 7, **caractérisé en ce qu'**on divise le film résultant en systèmes d'administration individuels par perforation, découpage transversal et/ou découpage longitudinal
